# EUROPEAN PATENT SPECIFICATION

(11) **EP 1 756 298 B1**
(45) Date of publication and mention of the grant of the patent: **09.06.2010**
(21) Application number: 05748372.9
(22) Date of filing: 09.06.2005
(51) Int. Cl.: C12Q 1/44

(54) **DIAGNOSIS AND PROGNOSIS OF WOUND INFECTION BY MEASUREMENT OF A PHOSPHOLIPASE A2 IN WOUND FLUID**
WUNDINFEKTIONSDIAGNOSE UND -PROGNOSE DURCH MESSUNG EINER PHOSPHOLIPASE A2 IN WUNDFLÜSSIGKEIT
DIAGNOSTIC ET PRONOSTIC D'INFECTION DE BLESSURES PAR LA MESURE D'UNE PHOSPHOLIPASE A2 DANS UN FLUIDE DE CETTE BLESSURE

(30) Priority: 11.06.2004 GB 0413078; 08.11.2004 US 625830 P
(43) Date of publication of application: 28.02.2007
(73) Proprietor: Systagenix Wound Management IP Co. BV., 1015 CS Amsterdam (NL)
(72) Inventor: SHAH, Faraia, Bradford BD9 6BE (GB); CLARK, Rachael, Skipton BD23 2DH (GB); TROTTER, Patrick, John, Leeds LS16 8BN (GB); WATT, Paul, William, Steeton BD20 6UN (GB); CULLEN, Breda, Mary, Skipton BD23 1HR (GB)
(74) Representative: James, Anthony Christopher W.P.
(86) International application number: PCT/GB2005/002262
(87) International publication number: WO 2005/121357

(56) References cited:
- WO-A-00/54052
- WO-A-95/05479
- WO-A-03/040406
- HAMOOD ABDUL N ET AL: "Production of extracellular virulence factors for Pseudomonas aeruginosa isolates obtained from tracheal, urinary tract, and wound infections" JOURNAL OF SURGICAL RESEARCH, vol. 61, no. 2, 1996, pages 425-432, XP002349625 ISSN: 0022-4804

## Description

The present invention relates to the diagnosis and prognosis of wound infection, by testing wound fluid for the presence of a marker which is present in an amount which is indicative of an inflammatory condition. The marker is a high molecular weight phospholipase A₂ (cPLA₂).

In mammals, injury triggers an organised complex cascade of cellular and biochemical events that result in a healed wound. Wound healing is a complex dynamic process that results in the restoration of anatomic continuity and function; an ideally healed wound is one that has returned to normal anatomic structure, function and appearance.

Chronically contaminated wounds all contain a tissue bacterial flora. These bacteria may be indigenous to the patient or might be exogenous to the wound. Closure, or eventual healing of the wound is often based on a physician's ability to control the level of this bacterial flora. Infection of wounds by bacteria delays the healing process, since bacteria compete for nutrients and oxygen with macrophages and fibroblasts, whose activity are essential for the healing of the wound. Infection results when bacteria achieve dominance over the systemic and local factors of host resistance. Infection is therefore a manifestation of a disturbed host/bacteria equilibrium in favour of the invading bacteria. This elicits a systemic septic response, and also inhibits the multiple processes involved in wound healing. Lastly, infection can result in a prolonged inflammatory phase and thus slow healing, or may cause further necrosis of the wound. The granulation phase of the healing process will begin only after the infection has subsided.

In clinical practice, a diagnosis of infection is based on the presence of local pain, heat, swelling, discharge and redness, although many clinical indicators, such as inflammation and discharge, have a low predictive value of infection in wounds. Definitive diagnosis is achieved by microbiological analysis of wound samples. Tissue biopsy provides the most accurate results, but this is an invasive procedure that is difficult to achieve for the mass of specimens required. Wound swabbing is the most common wound sampling method used in the UK although its clinical value has been questioned. Furthermore, microbiological diagnosis of wound infection can take 48 to 72 hours, which allows time for infection to further develop if first-line/best-guess treatment is not employed immediately.

There therefore remains a need in the art for a method for the early diagnosis and prognosis of wound infection, and for devices and wound dressings for use in carrying out such methods.

cPLA₂ are enzymes that hydrolyse the sn-2 position of membrane glycerophospholipids to liberate arachidonic acid. The activity of these enzymes is therefore important in the production of eicosanoids (e.g. prostaglandins and leukoriennes) and therefore associated with inflammation and host defence mechanisms. A detailed review can be found in Kudo and Murakami, Prostaglandins and other Lipid Mediators (2002) 68-69, 3-58.

There are essentially two groups of PLA₂: high molecular weight PLA₂ (cytosolic PLA₂) and low molecular weight PLA₂ (soluble PLA₂). High Mol. Wt. cytosolic cPLA₂ is normally located intracellularly and as such would not be expected to be present in wound fluid. There are three isoforms of this cPLA₂: α (1110kda), β (85kDa) and γ (60kDa) (See Kudo and Murakami, 2002). The low molecular weight soluble PLA₂'s (sPLA₂) are known to act intracellularly and, in contrast to cPLA₂, we have not found any evidence that the low molecular weight form (sPLA₂) is elevated in infected wound fluid.

WO03/101487 describes a method of assessing neural inflammatory disease in an animal by determining the level of phospholipase A₂ protein (including cPLA₂) in tissue or body fluid such as blood, plasma and cerebrospinal fluid. It does not appear to teach or suggest the association of cPLA₂ with extracellular wound fluid or as a marker of bacterial infection.

WO00/54052 describes an assay for detecting cPLA₂ in or on red blood cells, in particular for the diagnosis of diseases in which dysfunction of cell signalling systems involving highly unsaturated fatty acids are implicated. This reference does not appear to teach or suggest the testing of extracellular fluid for the presence of cPLA₂ to diagnose infection of a wound.

Funakoshi et al. in Pancreas (1991) vol. 6(5), pp 588-594 disclose that serum PLA₂ levels are elevated in the sera of patients with pancreatic diseases and that the measurement of serum PLA₂

Is useful for diagnosis and monitoring of pancreatitis. There is no teaching or suggestion of any association of cPLA2 with extracellular wound fluid or as a marker of bacterial wound infection.

Abdul N. Hamood et al. in Journal of Surgical Research, vol. 61(2), pages 425-432 (1996) describe the production of extracellular virulence factors, including phospholipase C, by *Pseudomonas Aeruginosa* isolates obtained from tracheal, urinary tract and wound infections.

WO-A-9505479 describes a novel assay for cPLA2 activity. There is no suggestion to apply the assay to wound fluid.

WO-A-03040406 describes the use of a fibronectin fragment, a neutrophil protease, or a macrophage protease, as a marker of bacterial infection in wound fluid.

In a first aspect, the present invention provides a method of diagnosing or prognosing a wound infection, the method comprising testing a sample of wound fluid which has been removed from the body for the presence or level of cPLA₂.

In a second aspect, the present invention provides a system for use in the diagnosis and treatment of wounds comprising: a wound dressing or biosensor comprising components of an assay system for testing wound fluid for the presence or level of cPLA₂; and a wound dressing comprising at least one antimicrobial agent, for application to the wound when the measured presence or level of cPLA₂ is indicative of wound infection; wherein the said assay system comprises an immunological binding partner for the cPLA₂.

The present invention relates to a marker of inflammation / infection that could be used as a target molecule in a new diagnostic / prognostic assay (e.g. laboratory based or point of care) to identify an inflammatory condition such as infection. The marker is a high molecular weight phospholipase A₂ (cPLA₂).

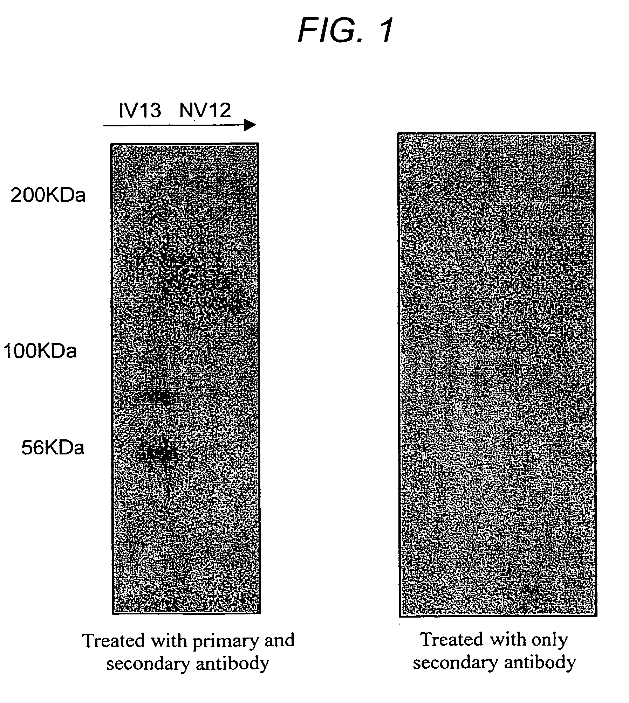

Western blot analysis of wound fluid from infected and non-infected patients shows that cPLA₂ and cPLA₂ fragments (three bands are present) are observed in infected wound fluid (IV 13) and no immunoreactivity to cPLA or cPLA₂ fragments was seen in the non-infected wound fluid (see attached figure 1). Based on the specificity of the antibody and the molecular weight it is believed that the three bands observed correspond to cPLA₂α (110kda), cPLA₂β (85kDa) and cPLA₂γ (60kDA).

cPLA₂ normally acts intracellularly and is not normally present in an wound environment. The observation that isoforms of this protein are present in infected but not in non-infected wound fluid is therefore novel and surprising. It is believed that the data presented herein represents the first demonstration that the cytosolic form of PLA₂ is even present in wound fluid.

The finding that cPLA₂ is present in infected fluid means that the differentiation between infected and basal levels is significant. cPLA₂ could therefore be used as a marker of inflammatory conditions such as infection and components for detecting or measuring the same could be incorporated in any type of diagnostic / prognostic kit (lab based (e.g. ELISA) or point of care (e.g. an antibody type kit similar to commercially available pregnancy kits).

The test on the wound fluid may be qualitative. Alternatively, a quantitative or semi-quantitative test for the marker (i.e. cPLA₂) may be performed. Thus, in one embodiment the concentration of the marker is measured.

The method is performed on wound fluid which has been removed from the body (e.g. as a clinical swab or as a fluid sample).

Various methods may be used to detect or measure the concentration of the marker. Suitable methods include those utilising chemical or enzyme-linked reactions, or immunological (e.g. ELISA, western blots), spectrophotometric, colorimetric, fluorimetric, or radioactive detection based techniques. In one embodiment a dip-stick type test is provided. Such a test could be used in the community and by the patient allowing easier and earlier diagnosis / prognosis.

For example, in the case of surface-exposed wounds, a clinical swab, dressing, "dipstick" or other biosensor device may be applied directly to the surface of the wound. The device should contain the components of the assay system for detecting the marker so that the assay reaction may itself proceed *in situ*. The device can then be removed from the wound and the signal measured by the appropriate means. In many cases, a physician may not actually require an accurate assessment of the precise concentration of the marker, but may just wish to know whether there is a sufficient concentration of the marker to warrant prophylactic or curative action as necessary. In these cases, visible assessment of the dressing may be sufficient to allow identification of the specific areas of infection. Unnecessary treatment of healthy granulating tissue can then be avoided.

A dressing that allows mapping of the infected areas of a wound will be preferable in certain instances. Diagnostic wound mapping sheets that could be adapted to the methods of the present invention are described in GB-A-2323166.

Immobilisation of reaction components onto a dipstick, wound mapping sheet or other solid or gel substrate offers the opportunity of performing a more quantitative measurement. For example, in the case of a reaction linked to the generation of a colour the device may be transferred to a spectrometer. Suitable methods of analysis will be apparent to those of skill in the art.

Immobilisation of the reaction components to a small biosensor device will also have the advantage that less of the components (such as antibody, enzyme and substrate) are needed. The device will thus be less expensive to manufacture than a dressing that needs to have a large surface area in order to allow the mapping of a large wound area.

Methods for the incorporation of the components of the assay reaction onto a clinical dressing, "dipstick", sheet or other biosensor are routine in the art. See for example Fägerstam and Karlsson (1994) Immunochemistry, 949-970.

Suitably, the methods employ an immunological binding partner for the cPLA₂. Such an assay system is employed in the system of the present invention. Suitable immunological binding partners include antibodies, including both polyclonal antibodies and monoclonal antibodies. Antibodies which may be employed in the present invention include Polyclonal antibody against c-terminal end of PLA2(abcam 9014); and monoclonal antibody to cPLA2 -US Biological Cat non. P4074-04 and are available commercially. The immunological binding partner may be immobilized or bound to a solid substrate in a device as described herein.

The detectable signal produced by the device is observable or measurable by a physical, chemical, or biological means known to those of skill in the art. A detectable signal may be a change in emission or absorbance of electromagnetic waves at a certain wavelength, hybridization or enzymatic reaction. In preferred embodiments, detectable signals are changes in colour when viewed under white light, or fluorescence when viewed under UV light. In certain embodiments, the device may comprise an electronic sensor, for example to detect color change or fluorescence and to provide a quantitative output thereof. The device may include an electronic sensor that can provide a quantitative output in digital form.

The device may further comprise a reference assay element for determining the total protein content of the sample, so that the measured level of cPLA₂ can be normalised to constant total protein level in order to increase accuracy.

In certain embodiments, the device for use in the present invention comprises or consists essentially of a wound dressing, dipstick or swab. In certain embodiments, the device comprises a housing containing one or more reagents and having an inlet provided therein for introduction of the sample. The housing may be at least partially transparent, or may have windows provided therein, for observation of an indicator region that undergoes a color or fluorescence change. In certain embodiments, the device operates on the lateral flow principle. That is to day, said device comprises a housing having an inlet for the sample and side walls defining a fluid lateral flow path extending from the inlet. By "lateral flow", it is meant liquid flow in which the dissolved components of the sample are carried, preferably at substantially equal rates, and with relatively unimpaired flow, laterally through the carrier. Suitably, the fluid flow path contains one or more porous carrier materials. The porous carrier materials are preferably in fluid communication along substantially the whole fluid flow path so as to assist transfer of fluid along the path by capillary action. Suitably, the porous carrier materials are hydrophilic, but preferably they do not themselves absorb water. The porous carrier materials may function as solid substrates for attachment of reagents or indicator moieties. In certain embodiments, the device further comprises a control moiety located in a control zone in said in said device, wherein the control moiety can interact with a component of the wound fluid sample to improve the accuracy of the device.

The size and shape of the carrier are not critical and may vary. The carrier defines a lateral flow path. Suitably, the porous carrier is in the form of one or more elongate strips or columns. In certain embodiments, the porous carrier is one or more elongate strips of sheet material, or a plurality of sheets making up in combination an elongate strip. One or more reaction zones and detection zones would then normally be spaced apart along the long axis of the strip. However, in some embodiments the porous carrier could, for example be in other sheet forms, such as a disk. In these cases the reaction zones and detection zones would normally be arranged concentrically around the center of the sheet, with a sample application zone in the center of the sheet. In yet other embodiments, the carrier is formed of carrier beads, for example beads made from any of the materials described above. The beads may suitably be sized from about 1 micrometer to about 1mm. The beads may be packed into the flow path inside the housing, or may be captured or supported on a suitable porous substrate such as a glass fiber pad.

It will be appreciated that the devices for use in the present invention may be adapted to detect at least one analyte in addition to the cPLA₂. This can be done by the use of several different reagents in a single reaction zone, or preferably by the provision in a single device of a plurality of lateral flow paths each adapted for detecting a different analyte. In certain embodiments, the plurality of lateral flow paths are defined as separate fluid flow paths in the housing, for example the plurality of lateral flow paths may be radially distributed around a sample receiving port. In some embodiments, the plurality of fluid flow paths are physically separated by the housing. In other embodiments multiple lateral flow paths (lanes) can be defined in a single lateral flow membrane by depositing lines of wax or similar hydrophobic material between the lanes.

The devices for use in the present invention may for example be incorporated into a bacterial sensing device of the following kind.

Briefly, the devices are lateral flow sensors for the detection of endogenous and/or microbial protease enzymes in wound fluid in order to ascertain the amount and type of bacterial infection. The devices comprise: a housing having an inlet for the sample and side walls defining a fluid flow path extending from the inlet, an indicator moiety that is bound to a solid substrate by means of a peptide linker moiety that is cleavable by the analyte enzyme, the solid substrate being located in a reaction zone of the fluid flow path; and a detector moiety located in a detection zone downstream from the reaction zone in the fluid flow path, wherein the detector moiety can interact with an indicator moiety that has been cleaved from the solid substrate to produce a detectable change in the detection zone.

An absorbent element may suitably be included in the devices for use in the present invention. The absorbent element is a means for drawing the whole sample through the device by capillary attraction. Generally, the absorbent element will consist of a hydrophilic absorbent material such as a woven or nonwoven textile material, a filter paper or a glass fiber filter.

The device may further comprise at least one filtration element to remove impurities from the sample before the sample undergoes analysis. The filtration device may for example comprise a microporous filtration sheet for removal of cells and other particulate debris from the sample. The filtration device is typically provided upstream of the sample application zone of the fluid flow path, for example in the inlet of the housing or in the housing upstream of the inlet.

Preferably, the devices for use in the present invention include a control moiety in a control zone of the device, wherein the control moiety can interact with a component of the wound fluid sample to improve the accuracy of the device. Suitably, the control zone is adapted to reduce false positive or false negative results. A false negative result could arise for various reasons, including (1) the sample is too dilute, or (2) the sample was too small to start with.

In order to address false negative mechanism , the control zone preferably further comprises a reference assay element for determining the total protease content or the total protein content of the sample, that is to say for establishing that the total protease content or the total protein content of the sample is higher than a predetermined minimum. It is possible to indicate the presence of protein by the use of tetrabromophenol blue, which changes from colorless to blue depending on the concentration of protein present. It is also possible to detect glucose (using glucose oxidase), blood (using diisopropyl-benzene dihydro peroxide and tetramethylbenzidine), leukocytes (using ester and diazonium salt). These may all be useful analytes for detection in the control zone for the reduction of false negatives.

The present invention may employ a diagnostic test system or kit comprising a diagnostic device as described above. The test system or kit may comprise, in addition to a diagnostic device, one or more components selected from: a color chart for interpreting the output of the diagnostic device, a sampling device for collecting a sample of a wound fluid from a wound, a wash liquid for carrying a sample of wound fluid through the device, and a pretreatment solution containing a reagent for pretreatment of the wound fluid sample.

Where present, the sampling device may comprise a swab or a biopsy punch, for example a shaft having a swab or biopsy punch attached thereto. Suitably, the diagnostic device includes a sample receiving port, and preferably the sample receiving port and the swab or biopsy punch comprise complementary fitting elements whereby the swab or biopsy punch can be secured to the device with the swab or biopsy punch received in the sample receiving port.

In certain embodiments the fitting element on the shaft may be located from 1mm to about 30mm from the base of the swab or the biopsy punch. This is consistent with the use of relatively small sample receiving port on the housing of the diagnostic device. The sample receiving port is typically located on an upper surface of the diagnostic device, and it is typically generally in the form of an upwardly projecting tube, open at the top and having the inlet to the fluid flow path located at the bottom of the tube.

The fitting element on the shaft may a tapered region of the shaft for forming an interference fit with the housing, for example it may appear as a truncated cone that is coaxial with the shaft and tapers towards the first end of the shaft. Or the whole shaft may have a diameter larger than that of the swab or biopsy punch, with a tapered region adjacent to the first end. In any case, the diameter of the tapered region where it engages with the housing is normally greater than the diameter of the swab or biopsy punch, so that the inlet port can enclose the swab or biopsy punch.

In other embodiments, the engagement element may comprise a snap-fitting projection for forming a snap-fit with one or more complementary projections on an inner surface of the housing, or a threaded projection for forming a screw fit with one or more complementary threads on an inner surface of the cap, or a Luer-lock type fitting.

The swab may be any absorbent swab, for example a nonwoven fibrous swab. Typically the diameter of the swab is about 2 to about 5mm, for example about 3mm. In certain embodiments, the swab may be formed from a medically acceptable open-celled foam, for example a polyurethane foam, since such foams have high absorbency and can readily be squeezed to expel absorbed fluids. The biopsy punch will typically be a stainless steel cylindrical punch of diameter about 1mm to about 10mm, for example about 3mm to about 8mm, suitably about 6mm.

In certain embodiments the shaft is hollow, whereby a fluid can be passed down the shaft from the second end to expel the biological sample from the swab or the biopsy punch into the diagnostic device. This helps to ensure that all of the sample passes through the device, thereby avoiding false negatives. The shaft may comprise a fitting at the second end for attachment of a syringe or other source of the fluid. In certain embodiments, the apparatus may comprise a reservoir of liquid attached to the second end of the shaft, for example a compressible bulb containing the liquid, which can be activated after use of the swab or biopsy punch. Suitable devices of this kind are described, for example in US-A-5266266. In other embodiments, the apparatus may comprise a plunger that can be pushed down the hollow bore of the shaft to expel fluid or other specimens from the swab or biopsy punch.

Another advantage of the hollow shaft is that, where the apparatus is a biopsy punch, the biopsy sample can more readily be pushed or blown out of the punch. The biopsy punch apparatus can further comprise a homogenizing tool that can be passed down the hollow shaft to homogenize a tissue sample in the biopsy punch. This step of homogenizing can be followed, if necessary, by passing liquid down the shaft from the second end to expel the homogenized tissue from the biopsy punch into the device for diagnostic analysis.

The swab or biopsy punch may be sterilized, and may be packaged in a microorganism-impermeable container. The diagnostic devices for use in the present invention may also be sterilized, but they may not, because the devices often do not come into contact with the patient being diagnosed.

The concentration of the marker may also be measured in an aqueous assay system. For instance, wound fluid may be extracted directly from the environment of the wound or can be washed off the wound using a saline buffer. The resulting solution can then be assayed for the concentration of the marker in, for example, a test tube or in a microassay plate.

Such a method will be preferable for use in cases in which the wound is too small or too inaccessible to allow access of a diagnostic / prognostic device such as a dipstick. This method has the additional advantage that the wound exudate sample may be diluted.

It will be clear that an aqueous assay system is more applicable to use in a laboratory environment, whereas a wound dressing containing the necessary reaction components will be more suitable for use in a hospital or domestic environment.

In certain embodiments according to this aspect, the method is adapted to determine whether the presence or level of cPLA₂ in a sample of wound fluid exceeds a predetermined minimum level which is characteristic of an infection. In other embodiments, method is adapted to determine whether the presence or level of cPLA₂ in a sample of wound fluid exhibits an increase over time that is indicative of the development of infection.

Preferably, the system according to this invention furter comprises a wound dressing that is substantially free from antimicrobial agents, for application to the wound when the measured presence or level of cPLA₂ is indicative of a non-infected wound. The system may be in the form of a kit, and the device and the wound dressing(s) may be packaged together in a single package.

The invention can avoid unnecessary application of antimicrobial agents to the wound, which is desirable because most antimicrobial agents are cytotoxic and interfere with wound healing, and also to avoid the development of resistant microorganisms.

The antimicrobial wound dressing used in this aspect of the invention comprises an effective amount of an antimicrobial agent, which may preferably be selected from the group consisting of antiseptics and antibiotics and mixtures thereof. Suitable antibiotics include peptide antimicrobials (e.g. defensins, Magainin, synthetic derivatives of them) tetracycline, penicillins, terramycins, erythromycin, bacitracin, neomycin, polymycin B, mupirocin, clindamycin and mixtures thereof. Suitable antiseptics include silver sulfadiazine, chlorhexidine, povidone iodine, triclosan, other silver salts and colloidal silver, sucralfate, quaternary ammonium salts and mixtures thereof.

The wound dressing materials used in this aspect of the invention may for example be provided in the form of beads, flakes, powder, and preferably in the form of a film, a fibrous pad, a web, a woven or non-woven fabric, a freeze-dried sponge, a foam or combinations thereof. In certain embodiments, the dressing material is selected from the group consisting of woven fabrics, knitted fabrics, and nonwoven fabrics, all of which may be made by conventional methods. In other embodiments, the material may comprise (or consist essentially of) a freeze-dried sponge or a solvent-dried sponge.

The wound dressing material may be in the form of a solid, or a semi-solid ointment or gel. Preferably, the wound dressing material comprises only up to 20% by weight, preferably less than 10% by weight of water. The relatively low water content improves the stability of the material and makes it possible to sterilize by heat or irradiation without loss of activity. The material may also contain 0-40% by weight, preferably 0-25% by weight of a plasticiser, preferably a polyhydric alcohol such as glycerol. All of the above percentages are on a dry weight basis.

Any type of wound may be diagnosed or treated using the methods of the present invention. For example, the wound may be an acute wound such as an acute traumatic laceration, perhaps resulting from an intentional operative incision. More usually the wound may be a chronic wound. Preferably, the chronic wound is selected from the group consisting of venous ulcers, pressure sores, decubitis ulcers, diabetic ulcers and chronic ulcers of unknown aetiology. The present invention may be used in the diagnosis or prognosis of human and non-human mammalian animals.

As used herein, the term wound fluid is meant to refer to the exudate that is secreted or discharged by cells in the environment of the wound. The term "wound fluid" herein refers to any wound exudate or other fluid (preferably substantially not including blood) that is removed from the wound surface by aspiration, absorption or washing. The term "wound fluid" does not normally refer to blood or tissue plasma remote from the wound site.

By testing wound fluid for the presence or level of cPLA₂, we include testing for cPLA₂α, cPLA₂β or cPLA₂γ.

### BRIEF DESCRIPTION OF DRAWINGS

Figure 1 shows the results of a Western blot for cPLA₂ in infected and non-infected wound fluid. NV and IV fluid diluted 1:10.

### MODES FOR CARRYING OUT THE INVENTION

### EXAMPLE 1

### Collection and treatment of wound fluid - removal of infected and non-infected wound fluid

All patients enrolled in the study had venous leg ulcers of at least 30 days duration and a surface area of at least 1cm². Patients were diagnosed as 'non-infected', normal appearance of wound, or 'infected' based on a minimum of 4 clinical signs and symptoms indicative of infection. Patients were excluded from the study if exposed bone with positive osteomyelitis was observed. Other exclusion criteria included concomitant conditions or treatments that may have interfered with wound healing and a history of non-compliance that would make it unlikely that a patient would complete the study. Wound fluids were collected from the patients following informed consent being given from all patients or their authorized representatives. The protocol was approved by the Ethics Committee at the participating study center prior to commencement of the study. The study was conducted in accordance with both the Declaration of Helsinki and Good Clinical Practice.

### Sample preparation

Total protein in each extracted wound fluid sample was determined using the Bradford protein assay, following manufacturers instructions for use of a Bio-Rad Protein Assay kit. All non-infected samples were then coded by NV followed by a specific number and the infected samples coded by IV followed by a number. Neat samples were diluted in PBS as 1:5 and 1:10 and then mixed by pipetting with reduced treatment buffer - 20ul wound fluid mixed with 5ul reduced treatment buffer and then boiled for 5mins in a water bath, to degrade proteins to fragments and produce reduced samples. (NB tops of the eppendorf tubes must be open). Following this treatment all samples were frozen in-20°C until required for western blot analysis.

### Western blotting

Wound fluid from non-infected patient identified as NV12 and an infected patient IV 13 was used. Broad range SDS page marker (Catalog number 161-0318 obtained from Biorad) was used as a standard and 10% Tris-HCl gels (Biorad) used. Samples were loaded in the gel and SDS page electrophoresis carried out at 60mA until the samples ran to the bottom of the gel. Gels were transferred onto nitrocellulose paper of pore size 0.45um (Biorad) at 200mA over 3hrs whilst immersed in ice. Following gel transfer, nitrocellulose paper was washed in PBS / 0.1 % Tween solution for 5 mins and then blocked in 5% milk for lhr at room temperature, RT, on shaker. Following another wash in PBS/Tween solution, one blot was treated with primary antibody (abcam one polyclonal antibody, abcam 9014) diluted in PBS/Tween solution and the second blot was treated with only PBS/Tween solution as a control. Both blots were left on the shaker overnight at RT. The polyclonal primary antibody was obtained from Autogen Bioclear UK (sc20105) at a concentration of 200ug/ml and diluted -100ul neat added to 9,900ul PBS/Tween solution. Following incubation with the primary antibody, blots were washed every 5 mins in PBS / Tween solution for 30mins and then treated to secondary antibody (anti - sheep HRP-linked antibody obtained from Sigma) for 1hr at RT on the shaker. The blots were then developed using the Opti4 CN kit (Biorad) for 5-10mins and the bands observed.

### Additional data

Figure 1 shows information from one infected and one non infected patient. To confirm this observation the wound fluid of five non-infected (NV2, 5, 9, 12 and 14) and five infected patients (IV 1, 4, 10, 13 and 22) was assessed using the method as described above.

These results demonstrated that no cPLA₂ was observed in the non infected patients and cPLA₂ was present in 4 out of five infected patients. This additional experiment confirms that cPLA₂ is a marker of wound infection.

It will be understood that the invention has been described by way of example only and modifications may be made whilst remaining within the scope of the invention as defined in the accompanying claims.

## Claims

1. A method of diagnosing or prognosing a wound infection, the method comprising testing a sample of wound fluid which has been removed from the body for the presence or level of cytosolic phospholipase A₂ (cPLA₂).

2. A method according to claim 1, wherein the cPLA₂ is cPLA₂α, cPLA₂β or cPLA₂γ.

3. A system for use in the diagnosis and treatment of wounds comprising:
a wound dressing or biosensor comprising components of an assay system for testing wound fluid for the presence or level of cPLA₂; and
a wound dressing comprising at least one antimicrobial agent for application to the wound when the measured presence or level of cPLA₂ is indicative of wound infection;
wherein the said assay system comprises an immunological binding partner for the cPLA₂.

4. A system according to claim 3, further comprising a wound dressing substantially free from antimicrobial agents, for application to the wound when the measured presence or level of cPLA₂ is indicative of a non-infected wound.

## Patentansprüche

1. Verfahren zur Diagnose oder Prognose einer Wundinfektion, wobei das Verfahren das Testen einer Wundflüssigkeitsprobe, die von dem Körper entnommen wurde, auf die Anwesenheit oder den Spiegel von cytosolischer Phospholipase A₂ (cPLA₂) umfaßt.

2. Verfahren nach Anspruch 1, wobei die cPLA₂ cPLA₂α, cPLA₂β oder cPLA₂γ ist.

3. System zur Verwendung in der Diagnose und Behandlung von Wunden, umfassend:
einen Wundverband oder Biosensor, umfassend Komponenten eines Assaysystems zum Testen von Wundflüssigkeit auf die Anwesenheit oder den Spiegel von cPLA₂, und
einen Wundverband, umfassend mindestens ein antimikrobielles Mittel, zur Aufbringung auf die Wunde, wenn die gemessene Anwesenheit oder der gemessene Spiegel von cPLA₂ eine Wundinfektion anzeigt,
wobei das Assaysystem einen immunologischen Bindungspartner für die cPLA₂ umfaßt.

4. System nach Anspruch 3, weiter umfassend einen Wundverband, der im Wesentlichen frei von antimikrobiellen Mitteln ist, zur Aufbringung auf die Wunde, wenn die gemessene Anwesenheit oder der gemessene Spiegel von cPLA₂ eine nicht infizierte Wunde anzeigt.

## Revendications

1. Procédé de diagnostic ou de pronostic d'infection d'une plaie, le procédé comprenant la recherche, dans un échantillon de fluide de la plaie prélevé sur l'organisme, de la présence ou de la concentration de phospholipase A₂ cytosolique (cPLA₂).

2. Procédé selon la revendication 1, dans lequel la cPLA₂ est de la cPLA₂α, de la cPLA₂β ou de la cPLA₂γ.

3. Système destiné à être utilisé dans le diagnostic et le traitement des plaies, comprenant :
un pansement ou biocapteur comprenant les composants d'un système de dosage pour rechercher, dans le fluide d'une plaie, la présence ou la concentration de cPLA₂ ; et
un pansement comprenant au moins un agent antimicrobien, destiné à être appliqué sur la plaie lorsque la présence ou la concentration de cPLA₂ mesurée révèle que la plaie est infectée ;
ledit système de dosage comprenant un partenaire de liaison immunologique de la cPLA₂.

4. Système selon la revendication 3, comprenant en outre un pansement sensiblement exempt d'agents antimicrobiens, destiné à être appliqué sur la plaie lorsque la présence ou la concentration de cPLA₂ mesurée révèle que la plaie n'est pas infectée.
